# EUROPEAN PATENT SPECIFICATION

(11) **EP 0 391 960 B1**
(45) Date of publication and mention of the grant of the patent: **17.08.1994**
(21) Application number: 89900771.0
(22) Date of filing: 08.12.1988
(51) Int. Cl.: C12N 5/10, C12N 15/86, A61L 27/00, A61L 33/00

(54) **GENETIC MODIFICATION OF ENDOTHELIAL CELLS**
GENETISCHE MODIFIZIERUNG VON ENDOTHELIALEN ZELLEN
MODIFICATION GENETIQUE DE CELLULES ENDOTHELIALES

(30) Priority: 11.12.1987 US 131926
(43) Date of publication of application: 17.10.1990
(73) Proprietor: WHITEHEAD INSTITUTE FOR BIOMEDICAL RESEARCH, Cambridge, MA 02142 (US); HOWARD HUGHES MEDICAL INSTITUTE, Bethesda, MD 20817 (US)
(72) Inventor: WILSON, James, M., Ann Arbor, MI 48103 (US); MULLIGAN, Richard, C., Cambridge, MA 02138 (US)
(74) Representative: Holdcroft, James Gerald, Dr.
(86) International application number: US8804383
(87) International publication number: WO8905345

(56) References cited:
- EP-A- 0 206 025
- WO-A-87/00201
- Proceedings of the National Academy of Sciences of the USA, vol. 81, no. 20, October 1984, (Washington, US), R.D. Cone et al; pp. 6349-6353
- Chemical Abstracts, vol. 110, 1988, (Columbus, Ohio, US), D.L. Mooradian; s. abstract 21757g, & Diss. Abstr. Int. B 1988, 49(4), 1099
- DNA, vol. 7, no. 9, 1988, Mary Ann Liebert, Inc., Publishers, R.W. Connors et al. pp. 651-661
- Science, vol. 243, 13 January 1989, J.A. Zwiebel et al. pp. 220-222

## Description

### Background

The endothelium is a single layer of flattened transparent cells which are joined edge to edge, with the result that they form a membrane of cells. Endothelial cells originate during development from the embryonic mesoblast or mesoderm. They occur on the free surfaces of serous membranes, in the anterior chamber of the eye and on the surface of the brain and spinal cord. In addition, they form the lining membrane of the heart, blood vessels and lymphatics.

It is possible, using methods developed in recent years, to attain interspecies genetic recombination. Genes derived from different biological classes are able to replicate and be expressed in a selected microorganism. Therefore, it is possible to introduce into a microorganism genes specifying a metabolic or synthetic function (e.g., hormone synthesis, protein synthesis, nitrogen fixation) which is characteristic of other classes of organisms by linking the genes to a particular viral or plasmid replicon.

Since the late 1970s, progress has been made toward the development of general methods for introducing cloned DNA sequences into mammalian cells. At the present time, however, there is a need for an effective method of stably introducing selected genetic material of interest into endothelial cells and enabling them to express it, thus producing the encoded protein or polypeptide.

### Summary of the Invention

The invention described herein relates to endothelial cells (e.g. human endothelial cells) for use in therapy, the cells containing stably incorporated genetic material encoding a product of interest, the cells being capable of expressing the genetic material to produce the encoded product.

The invention also covers the use of these endothelial cells for the manufacture of a medicament for use in therapy.

Endothelial cells of this invention have stably introduced in them genetic material of interest, which encodes a product (e.g., a protein or polypeptide) whose production in the endothelial cells is desired. The introduced genetic material is DNA or RNA which; (a) does not normally occur in endothelial cells, (b) normally occurs in endothelial cells but is not expressed in them at biologically significant levels, (c) normally occurs in endothelial cells and has been modified so that it is expressed in endothelial cells, or (d) can be modified to be expressed by endothelial cells, alone or in any combination thereof.

Endothelial cells of the present invention can also express genetic material encoding a selectable marker, thus providing a means by which cells expressing the incorporated genetic material are identified and selected for. Endothelial cells containing incorporated genetic material are referred to as transduced endothelial cells.

The invention also relates to a method of making the endothelial cells according to the invention, comprising the steps of (a) contacting cultured endothelial cells with media containing an infectious retrovirus having a recombinant genome comprised of the genetic material of interest and (b) maintaining the cultured endothelial cells and the media containing the infectious recombinant retrovirus under conditions appropriate for infection of the endothelial cells by the recombinant retrovirus.

Transduced endothelial cells of the present invention are particularly useful for improving prosthetic implants (e.g., vessels transplanted from one site in an individual to another; vessels implanted from another animal, such as pig, into an individual; vessels made of synthetic materials such as Dacron and polytetrafluoroethylene, which are used in vascular reconstructive surgery) and for providing constitutive delivery of polypeptides or proteins, useful in prevention and therapy or treatment, which are presently administered parenterally. The invention also relates to a lined prosthetic vessel comprising the endothelial cells of the invention, for implantation into a mammal and expression of the incorporated genetic material in the mammal.

There are many advantages to endothelial cells of the present invention. For example, they can be designed to improve the characteristics of endothelial cell-lined prosthetic implants by enhancing or improving the ability of endothelial cells to seed or bind to the inner surface of the implant; by modifying the endothelial cells used to line an implant so that they will grow; or by overcoming the problem, encountered with presently-available implants, of smooth muscle cell growth at the implant ends, which results in narrowing and, ultimately, closing off of the ends. In addition, the transduced endothelial cells of the present invention can be used to provide a drug, hormone or enzyme delivery system.

For example, prosthetic arterial grafts are often used to replace diseased arteries which perfuse vital organs or limbs. However, the currently available grafts are usually made of synthetic material and are subject to many complications, the worst of which is a high rate of re-stenosis or occlusion. Animal studies suggest that lining the graft with autologous endothelial cells prior to implantation may decrease, but not prevent, graft reocclusion with its attendant morbid consequences. Endothelial cells can be modified according to the method of the present invention in a way that improves their performance in the context of an implanted graft. Examples include secretion or expression of a transmembrane thrombolytic agent to prevent intraluminal clot formation, secretion of an inhibitor of smooth muscle proliferation to prevent luminal stenosis due to smooth muscle hypertrophy, and expression and/or secretion of an endothelial cell mitogen or autocrine factor to stimulate endothelial cell proliferation and improve the extent of rate of or duration of the endothelial cell lining of the graft lumen.

An additional application is to cover the surface of prosthetic heart valves with transduced endothelial cells in an attempt to improve the performance/longevity of the artificial valves.

A vascular implant lined with endothelial cells which include incorporated genetic material encoding a polypeptide of interest synthesizes the encoded polypeptide and thus serves as a continuous delivery system for that polypeptide. In this way, the hormone or other polypeptide is secreted directly into the bloodstream of the individual receiving the implant.

An important advantage is that the genetically engineered endothelial cells of the present invention can be used to administer therapeutic proteins (e.g., hormones, enzymes, clotting factors) which are presently administered intravenously, intramuscularly or subcutaneously. In addition, there is no need for extensive (and often costly) purification of the polypeptide before it is administered to an individual, as is generally necessary with an isolated polypeptide (e.g., insulin). Endothelial cells modified according to the present invention produce the polypeptide hormone as it would normally be produced.

Another advantage to the use of genetically engineered endothelial cells lining prosthetic arterial implants is that one can target the delivery of therapeutic levels of a secreted product to a specific organ or limb. The secreted product will be delivered to tissue perfused by the grafted artery in high concentrations, thereby achieving a desired effect to a targeted anatomical location. This product will then be diluted to nontherapeutic levels in the venous circulation during its return to the heart. For example, one or more vessels containing transduced endothelial cells of the present invention which produce an angiogenic factor can be implanted into the leg of an individual whose circulation is compromised (e.g., as a complication of diabetes mellitus) resulting in inadequate blood flow to the foot. This will result in a high concentration of the encoded product (angiogenic factor) close to the site of the implanted vessel, thus maximizing the therapeutic availability of the product. As the blood flows from the extremity, the concentration of angiogenic factor is reduced to nontherapeutic levels.

Another important advantage of the delivery system of this invention is that because it is a continuous delivery system, the fact that polypeptide hormones have very short half lives is not a limitation. For example, the half life of human growth hormone (HGH) is approximately 19 minutes, of parathyroid hormone, approximately 2½ to 5 minutes and, of native insulin (pure insulin), approximately 3 to 4 minutes.

Because genes can be introduced into endothelial cells using a retroviral vector, they can be "on" (subject to) the retroviral vector control; in such a case, the gene of interest is transcribed from a retroviral promoter. A promoter is a specific nucleotide sequence recognized by RNA polymerase molecules that start RNA synthesis. Alternatively, retroviral vectors having additional promoter elements (in addition to the promoter incorporated in the recombinant retrovirus) which are responsible for the transcription of the genetic material of interest, can be used. For example, a construct in which there is an additional promoter modulated by an external factor or cue can be used, making it possible to control the level of polypeptide being produced by the endothelial cells by activating that external factor or cue. For example, heat shock proteins are proteins encoded by genes in which the promoter is regulated by temperature. The promoter of the gene which encodes the metal-containing protein metallothionine is responsive to cadmium (Cd⁺⁺) ions. Incorporation of this promoter or another promoter influenced by external cues also makes it possible to regulate the production of the polypeptide by the engineered endothelial cells.

### Brief Description of the Drawings

Figure 1 is a schematic representation of a wild type murine leukemia virus (retroviral) genome.

Figure 2 is a schematic representation of retroviral vectors, each having a recombinant genome, useful in the present invention. Figure 2a is pLJ and Figure 2b is pEm.

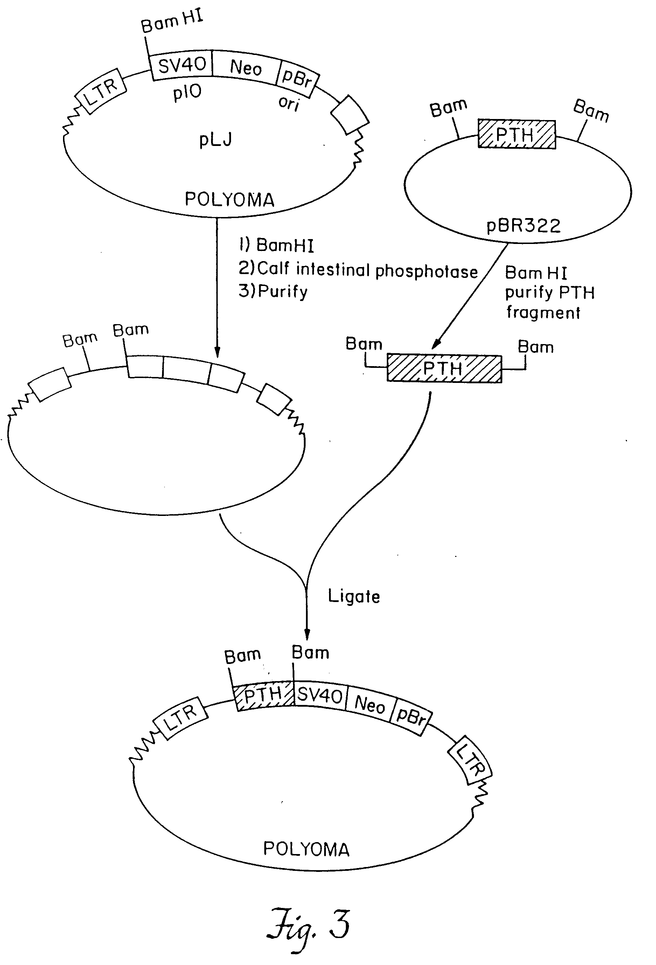

Figure 3 is a schematic representation of the construction of a recombinant retroviral vector, using the pLJ vector represented in Figure 2a and the human parathyroid hormone gene.

Figure 4 includes pictures of bovine aortic endothelial cells infected with a retrovirus that expresses low density lipoprotein receptor (LDLR) and analyzed for the uptake of fluorescent labeled LDL. Panel A - uninfected bovine aortic endothelial cells under phase contrast; Panel B - uninfected bovine aortic endothelial cells under fluorescent illumination; Panel C - infected bovine aortic endothelial cells under phase contrast; Panel D - infected bovine aortic endothelial cells under fluorescent illumination.

Figure 5 includes pictures of bovine aortic endothelial cells infected with a retrovirus that expresses B-galactosidase and analyzed for its expression using an in situ histochemical stain. Panel A - uninfected bovine aortic endothelial cells stained for beta-galactosidase activity; Panel B - infected unselected bovine aortic endothelial cells stained for beta-galactosidase activity.

### Detailed Description of the Invention

Genetic material of interest has been incorporated into endothelial cells and expressed in the resulting genetically engineered endothelial cells. Genetic material of interest incorporated into endothelial cells according to the method described can be any selected DNA of interest (e.g., all or a portion of a gene encoding a product of interest) or any selected RNA of interest. For example, it can be DNA or RNA which is present and expressed in normal endothelial cells; DNA or RNA which does not normally occur in endothelial cells; DNA or RNA which normally occurs in endothelial cells but is not expressed in them at levels which are biologically significant (levels sufficient to produce the normal physiological effects of the protein or polypeptide it encodes); DNA or RNA which occurs in endothelial cells and has been modified in such a manner that it can be expressed in such cells; and any DNA or RNA which can be modified to be expressed in endothelial cells, alone or in any combination thereof. This genetic material of interest is referred to herein as incorporated genetic material. Endothelial cells of the present invention express the incorporated genetic material. The incorporated genetic material (i.e., DNA or RNA) expressed by endothelial cells of the present invention is genetic material encoding a polypeptide or a protein of interest (genetic material of interest). Endothelial cells of the present invention can also include and express a gene encoding a selectable marker.

For example, genetic material of interest which is DNA normally present in and expressed by human endothelial cells can be incorporated into abnormal endothelial cells (e.g., which contain an abnormal quantity of the DNA or which contain but do not express such material adequately), with the result that they are able to produce the desired protein or polypeptide.

In another example, which has been carried out, genetic material encoding a hormone has been introduced into endothelial cells by exposing them to media that contains a virus having a recombinant genome (i.e., by infecting them). The media used is obtained by harvesting media in which producer cells (e.g., a Psi am or amphotropic producer) have been grown. That is, producer cells have been grown in tissue culture to a confluent density in Dulbecco's Modified Eagle's medium (DME) with 10% calf serum (CS) and penicillin and streptomycin. Fresh media is added and subsequently (e.g., approximately 12 hours later), the media is harvested. Approximately 10 ml of media is harvested from a 10 cm plate of confluent producer cells. The spent media (or viral stock) is filtered through a 0.45 micron Millipore filter to remove detached producer cells and is used immediately to infect cells or is stored at -70^{o} C. Media is removed from a subconfluent plate of endothelial cells (recipient endothelial cells) and quickly replaced with viral stock (e.g., 5 ml/10 cm. plate) containing 8 mcg/ml. of Polybrene (Aldrich). Subsequently (e.g., approximately 12 hours later), this is removed and replaced with fresh media. Thus, the media used is a viral supernatant. The recombinant genome of the infectious virus includes the genetic material of interest, which is incorporated into endothelial cells. The recombinant genome can also have genetic material encoding a dominant selectable marker.

As a result, endothelial cells were made which express a polypeptide not normally expressed by such cells at biologically significant levels and, optionally, a dominant selectable marker.

In particular, endothelial cells are exposed to media containing infectious virus produced in Psi am cells; the infectious virus contain a recombinant genome having the genetic material of interest. The recombinant genome in one instance includes genetic material encoding human parathyroid hormone (hPTH). Optionally, it can also include a gene encoding a dominant selectable marker (e.g., the neo gene which encodes neomycin resistance in bacteria and G418 resistance in mammalian cells). As a result, the endothelial cells are transduced -- that is, the genetic material of interest (in this case, DNA encoding hPTH and, optionally, the neo gene) is stably introduced into the endothelial cells. The transduced endothelial cells express the encoded hPTH and, if the neo gene is present, also express it, resulting in cells having the selectable trait.

Alternatively, endothelial cells are transduced as a result of exposure to media containing infectious virus in which the recombinant genome includes genetic material of interest (e.g., any gene of interest or a portion thereof, RNA of interest). As described below, endothelial cells have been transduced with a gene which codes for a secreted product (human parathyroid hormone or hPTH); a gene which codes for a membrane receptor (low density lipoprotein receptor or LDLR); and a gene coding for an intracellular enzyme (beta-galactosidase).

Endothelial cells expressing the incorporated genetic material are grown to confluence in tissue culture vessels; removed from the culture vessel in which they were grown; and introduced into or implanted into the body. They can be introduced into the recipient by one of several methods. For example, transduced endothelial cells can be seeded onto the lumen of a prosthetic vessel. The endothelial cells will then grow to confluence, covering the lumen of the vessel. They form the lining of what has been called a neointima (a well organized structure that resembles the intima and media of a native vessel (i.e., a covering of endothelial cells with deeper layers of smooth muscle cells).

Alternatively, they can be grafted onto a blood vessel in vivo through the use of a catheter. It is also possible they can be introduced into body cavities which are lined by serosal membranes, such as the peritoneal cavity, the pleural space, and the pericardial space. In this case, the endothelial cells would seed the serosal lining and secrete the product into the cavity. The product would then be absorbed via the lymphatic system.

Another example which has been carried out is the implantation into dogs of vascular grafts that have been seeded with autologous retrovirus-transduced endothelial cells. Replication-defective retroviruses with amphotropic host range have been used to stably introduce a reporter gene into the genome of the endothelial cells. The lac Z gene has been used as the reporter gene because its product of expression, beta-galactosidase, can be detected in-situ through the use of enzyme histochemical assays that stain the cells cytoplasm blue.

External jugular veins harvested from adult mongrel dogs were used as a source of endothelial cells which were plated in vitro during a 10-14 day period by two serial passages. Cells from each animal were divided into two aliquots and were either infected with a replication defective retrovirus or mock infected. Small diameter dacron grafts were seeded at subconfluent densities with endothelial cells by the autologous clot method and surgically implanted as carotid interposition grafts into the dog from which the cells were harvested; each dog received a graft seeded with the genetically modified cells and a contralateral graft seeded with MOCK infected cells. Five weeks after implantation, the grafts were harvested and analyzed.

Cells were enzymatically harvested from the luminal surface of a portion of the graft to permit a more detailed characterization. Primary cultures of cells were established and expanded in vitro for approximately 2-3 weeks prior to analysis. Genetically modified endothelial cells were identified in this population by Southern analysis and by the cytochemical assay for viral-expressed beta-galactosidase. The majority of cells harvested from the graft and expanded in vitro retained differentiated endothelial function (less than 95%). However, the proportion of cells that expressed viral directed beta-galactosidase or contained proviral sequences was consistently decreased 2-10 fold when compared to the cultures that were analyzed at the time of seeding. This disparity is due in part to the partial repopulation of grafts with endogenous cells by growth through interstices or from the anastomoses.

The results of this study demonstrate the feasibility of implanting vascular grafts seeded with autologous genetically modified endothelial cells. The transduced cells persist on the lumen of the graft for at least five weeks and the transferred gene continues to function.

### Isolation of Endothelial Cells

The isolation and maintenance of endothelial cells from capillaries and large vessels (e.g., arteries, veins) of many species of vertebrates has been well described in the literature. For example, McGuire and Orkin describe a simple procedure for culturing and passaging endothelial cells from large vessels of small animals. McGuire, R.W. and R.W. Orkin, Biotechniques, 5:546-554 (1987).

Frequently, calf aorta is the source of endothelial cells, as was the case in the initial work described herein. A typical protocol for isolation of endothelial cells from a large artery is described below. Sections of aortas freshly harvested are placed under sterile conditions, in a solution containing collagenase (e.g., 0.5 mg/ml) for 15-20 minutes at 37^{o}C. The aortas are then rinsed twice with tissue culture medium (e.g., RPMI 1640) and the lumenal sheet of endothelial cells is removed in complete medium (RPMI containing 15 mm Hepes, pH 7.4, penicillin/streptomycin and 20% fetal calf serum) by gentle agitation, according to the method of Booyse et al.. Booyse, F.M. et al., Thrombosis Diath. Haemorrh., 34:825-839 (1975). The cell patches are transferred to tissue culture flasks in complete medium.

The cells divide and eventually cover the plate; when the plate is confluent, the cells can be passaged using standard tissue culture techniques. The purity of the cultures is assessed by uptake of fluorescent labeled acetylated LDL which is specifically taken up by endothelial cells. If the cultures are not pure (i.e., contaminated with cells other than endothelial cells, such as smooth muscle cells or fibroblasts), endothelial cells are cloned by limiting dilution and expanded to yield pure cultures. The life span of endothelial cells is limited in culture but varies markedly, depending on the anatomical source and donor animal. Bovine aortic endothelial cells have been maintained in culture for at least 15-20 passages.

### Retroviral Vectors

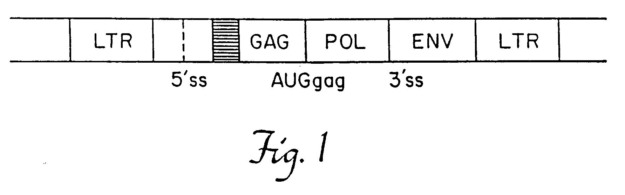

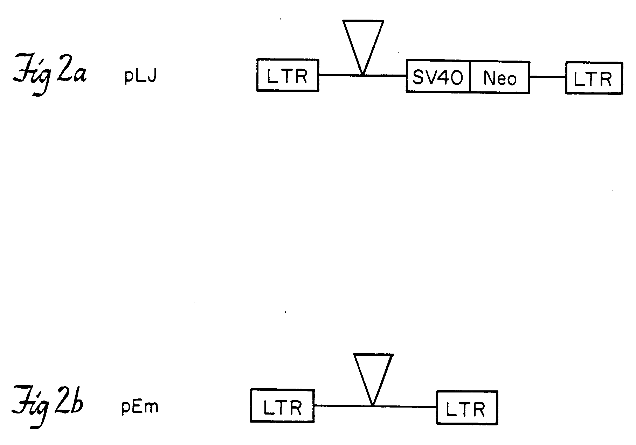

Retroviruses are RNA viruses; that is, the viral genome is RNA. This genomic RNA is, however, reverse transcribed into a DNA intermediate which is integrated very efficiently into the chromosomal DNA of infected cells. This integrated DNA intermediate is referred to as a provirus. As shown in Figure 1, the retroviral genome and the proviral DNA have three genes: the gag, the pol and the env, which are flanked by two long terminal repeat (LTR) sequences. The gag gene encodes the internal structural (nucleocapsid) proteins; the pol gene encodes the RNA-directed DNA polymerase (reverse transcriptase); and the env gene encodes viral envelope glycoproteins. The 5' and 3' LTRs serve to promote transcription and polyadenylation of virion RNAs.

Adjacent to the 5' LTR are sequences necessary for reverse transcription of the genome (the tRNA primer binding site) and for efficient encapsidation of viral RNA into particles (the Psi site). Mulligan, R.C., In: Experimental Manipulation of Gene Expression, M. Inouye (ed), 155-173 (1983); Mann, R., et al., Cell, 33:153-159 (1983); Cone, R.D. and R.C. Mulligan, Proceedings of the National Academy of Sciences, U.S.A., 81:6349-6353 (1984).

If the sequences necessary for encapsidation (or packaging of retroviral RNA into infectious virions) are missing from the viral genome, the result is a cis defect which prevents encapsidation of genomic RNA. However, the resulting mutant is still capable of directing the synthesis of all virion proteins. Mulligan and co-workers have described retroviral genomes from which these Psi sequences have been deleted, as well as cell lines containing the mutant stably integrated into the chromosome. Mulligan, R.C., In: Experimental Manipulation of Gene Expression, M. Inouye (ed), 155-173 (1983); Mann, R., et al., Cell, 33:153-159 (1983); Cone, R.D. and R.C. Mulligan, Proceedings of the National Academy of Sciences, U.S.A., 81:6349-6353 (1984). The teachings of these publications are incorporated herein by reference.

As described by Mulligan and co-workers, the Psi 2 cell line was constructed in the following manner: A mutant murine leukemia virus (MuLV) genome in which the region or the genome implicated in the encapsidation of viral RNA into virions is deleted (the Psi sequence in Figure 1) was constructed. This genome was stably introduced in NIH3T3 cells by DNA cotransformation and stable transformants that produced all of the viral proteins used for encapsidation, yet budded noninfectious particles, were isolated. The mutant of MuLV was constructed by deleting 351 nucleotides from an infectious proviral DNA clone between the putative env mRN4 5' splice site and the AUG that initiates the coding sequence for Pr 65^{gag}. The deletion was made from a Bal 1 site to a Pst 1 site and a Hind III site was generated at the point of deletion.

pMOV. (pMOVPsi⁻) was constructed as follows: Three purified DNA fragments were ligated together to construct pMOV Psi⁻. The first was obtained by digesting pMOV Psi⁺ with Xho I to completion, followed by partial digestion with Eco RI. Chumakov, I. et al., Journal of Virology, 42:1088-1098 (1982). The fragment extending from the Xho I site at 2.0 U in MuLV, through the 3' LTR, 3' mouse flanking sequence, all of pBR322, and ending at the Eco RI site was purified from an agarose gel after electrophoretic separation. Vogelstein, B. and D. Gillespie, Proceedings of the National Academy of Sciences, USA, 761:615-619 (1979). The second fragment was obtained by digestion of pMOV Psi⁺ with Bal I to completion followed by purification of the fragment extending from the Bal I site in pBR322 through 5' mouse flanking sequence and 5' LTR to the Bal I site located at 0.7 U of MuLV. Hind III linkers (Collaborative Research) were then blunt-ligated to this fragment with T4 DNA ligase, and the fragment was digested with excess Hind III and Eco RI. The LTR-containing fragment was purified from an agarose gel after electrphoretic separation. The third fragment present in the final ligation reaction was obtained from pSV2-gag/pol where the gag/pol region of MuLV had been subcloned into pSV2. Mulligan, R.C. and P. Berg, Science, 209:1422-1427 (1980). pSV2-gag/pol was digested to completion with Xho I and Hind III and the fragment extending from the Hind III site (changed from the Pst I site at 1.0 U of MuLV) to the Xho I site at 2.0 of MuLV was purified from an agarose gel following electrophoretic separation. These three DNA fragments were then mixed in equimolar amounts at a total DNA concentration of 50 ug/ml. in ligase buffer (50 mM Tris-HCl [pH 7.8], 10 mM MgCl2, 20 mM dithiothreitol, 1.0 mM ATP, 50 ug/ml. bovine serum albumin) and incubated with T4 DNA ligase for 18 hr. at 15^{o}C. E. coli HB101 was transfected with the ligated DNA, and ampicillin resistant transfectants were obtained. The plasmid DNA obtained from a number of transformants was screened for the desired structure by digestion with appropriate restriction endonucleases and electrophoresis through agarose gels. Davis, R.W. et al., Methods in Enzymology, 65:404-411 (1980).

Cell lines containing the Psi⁻ mutant stably integrated into the chromosome were made by cotransfection of pMOV-Psi⁻ and pSV2gpt, a SV40 hybrid vector capable of XG PRT expression. Mulligan, R.C. and P. Berg, Science, 209:1422-1427 (1980). Cells from gpt⁺ colonies obtained in this way were cloned and established into three lines: Psi-1, Psi-2, and Psi-3.

The Psi 2 cell line described by Mulligan and co-workers was created by transfecting NIH 3T3 endothelial cells with pMOV-Psi⁻, which is an ecotropic Moloney murine leukemia virus (Mo-MuLV) clone. pMOV-Psi⁻ expresses all the viral gene products but lacks the Psi sequence, which is necessary for encapsidation of the viral genome. pMOV-Psi⁻ expresses an ecotropic viral envelope glycoprotein which recognizes a receptor present only on mouse (and closely related rodent) cells.

Another cell line is the Psi am line, which are Psi-2-like packaging cell lines. These Psi-am cell lines contain a modified pMOV-Psi-genome, in which the ecotropic envelope glycoprotein has been replaced with envelope sequences derived from the amphotropic virus 4070A. Hartley, J.W. and W.P. Rowe, Journal of Virology, 19:19-25 (1976). As a result, they are useful for production of recombinant virus with amphotropic host range. The retrovirus used to make the Psi am cell line has a very broad mammalian host range (an amphotropic host range) and can be used to infect human cells. If the recombinant genome has the Psi packaging sequence, the Psi-am cell line is capable of packaging recombinant retroviral genomes into infectious retroviral particles. Cone, R. and Mulligan, R., Proceedings of the National Academy of Sciences, USA, 81:6349-6353 (1984).

The retroviral genome has been modified by Cone and Mulligan for use as a vector capable of introducing new genes into cells. As shown in Figure 2, the gag, the pol and the env genes have all been removed and a DNA segment encoding the neo gene has been inserted in their place. The neo gene serves as a dominant selectable marker. The retroviral sequence which remains part of the recombinant genome includes the LTRs, the tRNA binding site and the Psi packaging site. Cepko, C. et al., Cell, 37:1053-1062 (1984).

Additional vector constructions which have been used in producing transduced endothelial cells of the present invention are represented in Figure 2 and are described in detail below.

pLJ. The characteristics of this vector have been described in Korman, A.J. et al., Proceedings of the National Academy of Sciences, USA 84:2150 (1987). This vector is capable of expressing two genes: the gene of interest and a dominant selectable marker, such as the Neo gene. The gene of interest is cloned in direct orientation into a BamHI/Sma1/Sal1 cloning site just distal to the 5' LTR, while, the Neo gene is placed distal to an internal promoter (from SV40) which is farther 3' than is the cloning site (is located 3' of the cloning site). Transcription from PLJ is initiated at two sites: 1) the 5' LTR, which is responsible for expression of the gene of interest and 2) the internal SV40 promoter, which is responsible for expression of the neo gene. The structure of pLJ is represented in Figure 2a.

Vector pLJ is represented in Figure 2a. In pLJ, the genetic material of interest is inserted just following the 5' LTR. Expression of this genetic material is transcribed from the LTR and expression of the neo gene is transcribed from an internal SV40 promoter.

pEm. In this simple vector, the entire coding sequence for gag, pol and env of the wild type virus is replaced with the gene of interest, which is the only gene expressed. The components of the pEm vector are described below. The 5' flanking sequence, 5' LTR and 400 bp of contiguous sequence (up to the BamHI site) is from pZIP. The 3' flanking sequence and LTR are also from pZIP; however, the ClaI site 150 bp upstream from the 3' LTR has been ligated with synthetic BamHI linkers and forms the other half of the BamHI cloning site present in the vector. The HindIII/EcoR1 fragment of pBR322 forms the plasmid backbone. This vector is derived from sequences cloned from a strain of Moloney Murine Leukemia virus. An analogous vector has been constructed from sequences derived from the myeloproliferative sarcoma virus. The structure of pEM is represented in Figure 2b.

Vectors without a selectable marker can also be used to transduce endothelial cells with genetic material of interest. Such vectors are basically simplifications of the vectors previously described, in which there is such a marker. Vector pEm is represented in Figure 2b; as represented, the main components of the vector are the 5' and 3' LTR, and the genetic material of interest, inserted between the two LTRs.

### Introduction of Genetic Material into Endothelial cells and Assessment of Expression of the Genetic Material

A cell line which produces recombinant amphotropic retrovirus having a recombinant genome is used to infect endothelial cells. As described above, the recombinant genome can include a variety of components, but in general is comprised of two LTRs and, in place of the gag, the pol and the env sequences, a second promoter sequence. In some cases, it also includes a gene encoding a selectable marker (e.g., neo).

Viral stocks, to be used in introducing genetic material of interest into endothelial cells, are harvested, as described above, supplemented with 8 micrograms per ml. (mcg/ml.) of Polybrene (Aldrich) and added to the culture of endothelial cells. If the titer of the virus is high (e.g., approximately 10⁶ cfu per ml.), then virtually all endothelial cells will be infected and no selection (e.g., of endothelial cells into which the vector, including the recombinant genome, has been introduced) is required. If the titer is very low, then it is necessary to use a retroviral vector that has a selectable marker, such as neo or his. If a selectable marker is used, after exposure to the virus, the cells are grown to confluence and split into selective media (e.g., media containing G418 if the selectable marker is neo, media containing histidinol and no histidine if the selectable marker is his).

The neo gene is a bacterial gene derived from the transposon Tn5, which encodes neomycin resistance in bacteria and resistance to the antibiotic G418 in mammalian cells. This neo gene acts as a dominant selectable marker; its presence in a mammalian cell converts the cell into one which will grow in the presence of G418. (If it is not present, the cell dies in the presence of G418.) As a result, the presence of this gene in a mammalian cell can be determined by culturing cells in media which contains G418. The recombinant retrovirus having this recombinant genome is referred to as the neo virus.

The recombinant retroviral vectors having the neo gene also have a cloning site. As a result, genetic material of interest can be introduced into the vector, incorporated into endothelial cells and expressed by endothelial cells transduced with the recombinant retrovirus (referred to as endothelial cells having incorporated genetic material).

It should be possible to express virtually any gene of interest in endothelial cells by means of a retroviral vector. Retroviral vectors that express genes that encode three different classes of proteins have been constructed: a secreted hormone polypeptide (human PTH, or hPTH), a membrane receptor (receptor for LDL, or LDLR), and an intracellular enzyme (beta-galactosidase). Efficient expression of the recombinant viruses when incorporated into endothelial cells has been demonstrated and is described in detail in the examples.

### Introduction of Genetic Material Encoding Other Proteins or Polypeptides

Genes encoding other proteins or polypeptides can also be introduced into endothelial cells by means of an appropriate retroviral vector. For example, a gene encoding human growth hormone (HGH) or a gene encoding insulin can be introduced into endothelial cells. Such genes can be introduced into endothelial cells, alone or in combination with a gene encoding a selectable marker, such as the neo gene. HGH is a polypeptide of about 29,000 Daltons which is normally secreted only by the hypothalamus. Insulin is a polypeptide which regulates glucose levels in the bloodstream.

These genes, as well as others can be introduced into endothelial cells in the same manner as described above for the hPTH gene and the resulting transduced endothelial cells can be implanted into or applied to an appropriate site on the body.

### Use of Other Dominant Selectable Markers in the Introduction of Genetic Material Encoding Polypeptides

It is also possible to use dominant selectable markers other than the neo gene to introduce genetic material into endothelial cells. For example, the His D gene can be used for this purpose. The His D gene is a bacterial gene from Salmonella and encodes histidinol dehydrogenase, a polypeptide which converts histidinol to histidine. Histidine is an essential amino acid; histidinol is an alcohol analogue of histidine and can be converted to histidine under the proper metabolic conditions. If cells are grown in media containing histidinol but lacking histidine, those cells having the His D gene can convert histidinol to histidine. Because histidine is essential to their function, those cells which have the His D gene (and thus can make histidine) will survive and those lacking the gene will not.

A retrovirus vector having the His D gene has been used to infect keratinocytes. The keratinocytes containing His D gene were selected by growing these cells in media lacking histidine but containing histidinol. As expected, keratinocytes having the His D gene formed colonies and grew to confluence; those lacking the gene did not. In fact, such cells occurred at a much higher frequency than those in which the neo gene was included. These same techniques are useful in selecting endothelial cells containing DNA of interest.

As a result of this work, it is also possible to use independent dominant selectable markers (e.g., the neo gene and the His D gene) to incorporate new genetic material into endothelial cells. In the case of polypeptides which have two different subunits, for example, separate dominant selectable markers can be used to introduce the genetic information encoding the two subunits. In addition, two or more dominant selectable markers can be used in the case of polypeptides which need to be specifically cleaved or processed in order to become active (e.g., insulin and parathyroid hormone). A gene encoding the necessary processing enzyme can be introduced along with the gene encoding the polypeptide hormone requiring such processing. This would enable endothelial cells to process that polypeptide hormone.

### Other Vehicles for the Introduction of Genetic Material of Interest into Endothelial cells

It is also possible to use vehicles other than retroviruses to genetically engineer or modify endothelial cells. Genetic information of interest can be introduced into endothelial cells by means of any virus which can express the genetic material of interest in such cells. For example, SV40, herpes virus, adenovirus and human papilloma virus can be used for this purpose.

It is also possible to introduce genetic material of interest into endothelial cells in such a manner that it is not incorporated stably into the recipient cells, but is expressed episomally (remains distinct or separate from the recipient cell genome).

### Uses of Endothelial Cells Having Incorporated Genetic Material

### Improvement of Performance of Vascular Grafts or Implants

Many important disease states involve stenosis or occlusion of arteries that supply vital organs. The pathologic mechanism most commonly implicated in such disease states is atherosclerosis. Examples include angina pectoris and myocardial infarction due to coronary artery disease; transient ischemic attacks and strokes due to cerebral vascular disease; renal vascular hypertension, and ultimately, renal failure due to renal artery stenosis; and claudication of the lower extremities, which is caused by vascular disease of peripheral arteries and, in its most severe form, can result in amputation. Unless the agents that predispose an individual to atherosclerotic lesions are eliminated (e.g., hypertension, cigarette smoking, and hypertension), the natural history of these disease states is usually progression of the atherosclerotic lesions, resulting in permanent damage or death.

An accepted and widely used therapeutic approach to advanced atherosclerotic disease is to bypass the site of major stenosis or occlusion with a prosthetic vessel made of synthetic material, such as Dacron. More than 350,000 vascular grafts are implanted each year. One major problem with this approach is that the prosthetic vessel is extremely thrombogenic (i.e., it has the propensity to develop clots), which leads to a very high rate of re-stenosis. It has been possible to reduce this problem by seeding the lumen of the prosthetic vessels with autologous endothelial cells; grafts lined with endothelial cells are presumably less thrombogenic. It is in this setting that modified endothelial cells would be particularly useful.

Endothelial cells can be genetically modified according to the method of the present invention to improve their performance in the context of an endothelial cell-lined prosthetic implant. Existing protocols for using endothelial cell-lined prosthetic implants are complicated by several significant technological problems, most of which can be overcome through the use of genetically engineered endothelial cells.

First, it is impossible to achieve a confluent layer of endothelial cells lining the graft when cells are seeded at subconfluent density. This is due, in part, to the limited proliferative capacity of endothelial cells in this milieu. Furthermore, limited quantities of available autologous endothelial cells preclude the seeding of grafts at confluent densities (using currently available technology) in most relevant clinical situations. However, endothelial cells can be genetically modified to enhance their proliferative capacity by infecting them with a retrovirus which expresses an endothelial cell mitogen, such as endothelial cell growth factor. It may be possible to initially seed an implant with transduced endothelial cells at subconfluent densities and subsequently achieve a confluent lining in vitro or in vivo. Many applications of endothelial cell-lined prosthetic implantation which have been impossible due to limited quantities of autologous cells may now be feasible. In addition, the modified endothelial cells may exhibit a selective advantage over competing cells (such as smooth muscle cells or endogenous endothelial cells), thereby preventing overgrowth of the transduced endothelial cells by the competing cells.

An additional problem with endothelialized implants is that the lumen of the prosthetic vessel undergoes progressive narrowing due to the proliferation of smooth muscle cells between the wall of the prosthesis and the luminal surface. One way in which this can be prevented is to introduce into the endothelial cells a gene that secretes a product which inhibits the growth of smooth muscle cells. Many types of autocrine:paracrine growth factors that control proliferation of mesenchymal and/or epithelial cells have recently been identified and their genes cloned. Such genes can be introduced into endothelial cells using the method of the present invention. The resulting transduced endothelial cells produce the cell growth inhibitor.

A further technical problem of endothelialized implant protocols is that the binding (plating) efficiency of the endothelial cells to the prosthetic graft is relatively low. Previous attempts to improve this have been directed at modifying the composition of the graft surface and have been of limited success. Using the method of the present invention, it is possible to introduce into endothelial cells a gene which codes for a membrane receptor. The lumen of the prosthetic vessel can then be coated with the ligand for the receptor, thereby facilitating binding of endothelial cells to the luminal surface through the membrane receptor/ligand interaction.

Genetically engineered endothelial cells of the present invention can be used to decrease the thrombogenicity of endothelial cell-lined prosthetic grafts. The mechanism of clot formation is believed to be platelet adhesion followed by deposition of fibrin and propagation of clot. This could be minimized or possibly eliminated by seeding the grafts with genetically engineered endothelial cells that secrete a thrombolytic agent (e.g., an agent which dissolves clots, such as tissue plasminogen activator (TPA) or streptokinase). An even more effective approach would be to design a modified form of TPA which attaches to the endothelial cell membrane, rather than being secreted. In this case, the thrombolytic agents would be concentrated in the vessel lumen, where they would have the greatest effect.

### Use of Modified Endothelial Cells to Deliver a Product to a Limb or Organ

This invention can also be used to introduce into endothelial cells genes that secrete factors which would be beneficial to the limb or organ perfused by the artery containing the prosthesis. For example, a common clinical problem is the presence of extensive narrowing of small vessels distal to the site of the prosthetic vessel. This is characteristic of the vascular disease associated with diabetes mellitus. Revascularization of the larger vessels with implants leads to incomplete reconstitution of perfusion to the affected limb or organ. One could effect increased vascular flow to the compromised tissue by promoting the development of collateral circulation from contiguous tissue which is not compromised. This occurs naturally to a limited extent. However, the whole process of collateralization could be accelerated and more fully developed if endothelial cells in the affected area secrete a factor or factors that promote angiogenesis. The genes for many of these angiogenic factors have been cloned and can be introduced into endothelial cells (e.g., using a retrovirus in which the genome includes the gene of interest). The delivery of the angiogenic factor(s) would be targeted to the organ or limb supplied by the artery containing the graft. The effective concentration of the factor would be relatively high in the affected tissue, but low in general circulation because it would be diluted in the venous circulation upon its return to the heart. Thus, it should be possible to obtain a therapeutic effect in the diseased organ or limb without systemic side effects.

Another way of further promoting vascular flow to a compromised organ or limb is to maximally dilate all afferent vessels. Attempts to do this with oral or parenteral medicines have resulted in little therapeutic benefit, accompanied by many systemic side effects. This is caused, in part, by the fact that the vasodilator is not targeted to the appropriate tissue. Endothelial cells engineered to secrete a potent vasodilator such as atrial naturetic factor is an alternative approach. In this application, the affected organ or limb can be perfused with arterial blood which contains a very high concentration of a vasodilator. This results in an increase in the overall vascular perfusion. However, the vasodilator is diluted to nonpharmacologic levels upon return to the heart, thereby obviating the many systemic side effects of vasodilators that occur when administered in a nonselective manner.

### Use of Modified Endothelial Cells as a Delivery System

The present invention makes it possible to genetically engineer endothelial cells in such a manner that they produce a selected protein or polypeptide, such as selected polypeptides and proteins not normally produced in endothelial cells in biologically significant amounts, and secrete them into the bloodstream or other area of the body (e.g., the central nervous system). The endothelial cells formed in this way can serve as a continuous drug delivery system to replace present regimens, which require periodic administration (by ingestion, injection, etc.) of the needed substance.

For example, it can be used to provide continuous delivery of insulin, which, at the present time, must be isolated from the pancreas, extensively purified and then injected into the body by those whose insulin production or utilization is impaired. In this way, insulin can be introduced into the body via a continuous drug delivery system and, as a result, there would be no need for daily injections of insulin.

Genetically engineered endothelial cells can also be used for the production of clotting factors. Hemophiliacs lack a protein called Factor VIII, which is involved in clotting. Factor VIII is now administered by injection. Endothelial cells having genes encoding Factor VIII can be used to make an implant in which they produce Factor VIII; as an implant, the tissue would secrete the factor into the bloodstream.

Incorporation of genetic material of interest into endothelial cells can be particularly valuable in the treatment of inherited disease and the treatment of acquired disease. In the case of inherited diseases, this approach is used to provide genetically modified endothelial cells and other cells which can be used as a metabolic sink. That is, such endothelial cells would serve to degrade a potentially toxic substance. For example, transduced endothelial cells used in treating urea cycle disorders. Endothelial cells of the present invention can also be used in the treatment of genetic diseases in which a product (e.g., an enzyme or hormone) normally produced by the body is not produced or is made in insufficient quantities. Here, endothelial cells transduced with a gene encoding the missing or inadequately produced substance can be used to produce it in sufficient quantities. For example, this can be used in producing alpha-1 anitrypsin. It can also be used in the production of Factor VIII and Factor IX, and thus would be useful in treating hemophilia.

There are many acquired diseases for which treatment can be provided through use of genetically engineered endothelial cells (i.e., endothelial cells transduced with genetic material of interest). For example, such cells can be used in treating anemia, which is commonly present in chronic disease and often associated with chronic renal failure (e.g., in hemodialysis patients). In this case, endothelial cells having incorporated in them a gene encoding erythropoietin would correct the anemia by stimulating the bone marrow to increase erythropoiesis (i.e. production of red blood cells).

Endothelial cells of the present invention can also be used to administer a low dose of tissue plasminogen activator as an activator to prevent the formation of thrombi. In this case, endothelial cells having incorporated genetic material which encodes TPA would be transplanted into an individual in whom thrombus prevention is desired. This would be useful, for example, as a prophylactic against common disorders such as coronary artery disease, cerebrovascular disease, peripheral vascular occlusive disease, vein (e.g., superficial) thrombosis, such as seen in pulmonary emboli, or deep vein thrombosis. Endothelial cells which contain DNA encoding calcitonin can be used in the treatment of Paget's Disease, a progressive, chronic disorder of bone metabolism. Present treatment relies on subcutaneous administration of calcitonin.

Endothelial cells engineered to produce and secrete interleukins (e.g., IL-1, IL-2, IL-3) can be used in several contexts. For example, the result of some of the therapies now used (e.g., chemotherapy) is induction of neutropenia (the presence of abnormally low numbers of neutrophils in the blood), often caused by direct suppression of the bone marrow. For example, use of virtually all the chemotherapeutic agents, as well as AZT, used in the treatment of (AIDS) Acquired Immune Deficiency Syndrome, results in neutropenia. This condition results in numerous life-threatening infections. In these cases, administration of, for example, IL-3 through implantation of endothelial cells which contain genetic material encoding IL-3 can be used to increase the neutrophil count. In addition, the administration of thrombopoietin, which stimulates the production of platelets, can be used in the treatment of numerous conditions in which platelet count is low. In this case, endothelial cells transduced with the gene for thrombopoietin can be applied to/implanted in an individual; production and secretion of the encoded product will result in stimulation of platelet production.

Another related application of endothelial cells having incorporated genetic material is in the treatment of AIDS. Interleukin 2 and Interleukin 3, which stimulate the immune system, are potentially valuable in the treatment of AIDS. They could be delivered by a vessel implant having endothelial cells which have been genetically engineered to produce these two polypeptides (which are now administered by periodic injection).

Another use of the present invention is in the treatment of enzyme defect diseases. In this case the product encoded by the gene introduced into endothelial cells is not secreted (as are hormones); rather, it is an enzyme which remains inside the cell. There are numerous cases of genetic diseases in which an individual lacks a particular enzyme and is not able to metabolize various amino acids or other metabolites. The correct genes for these enzymes could be introduced into an endothelial cell implant; the implant would then carry out that metabolic function. For example, there is a genetic disease in which those affected lack the enzyme adenosine deaminase. This enzyme is involved in the degradation of purines to uric acid. It might be possible, using the present invention, to produce an implant capable of producing the missing enzyme at sufficiently high levels to detoxify the blood as it passes through the area at which the implant is applied.

The present invention also has veterinary applications. It can be used, for example, in delivering substances such as drugs (e.g., antibiotics) and hormones to animals, which would otherwise be provided by being incorporated into their feed, added to their water or injected periodically (e.g., daily or less frequently). Use of the modified endothelial cells of the present invention has the advantage that the tissue formed of the modified cells can be applied to the animal and will provide quantities of the encoded protein on an ongoing basis, thus eliminating the need for daily/periodic administration of the substance.

The present invention will now be illustrated by the following examples, which are not intended to be limiting in any way.

### EXAMPLE 1 Production of Human Parathyroid Hormone in Transduced Endothelial Cells

At the BamHI cloning site of pLJ, genetic material of interest can be inserted. The genetic material of interest can be DNA as described above.

In particular, a copy of the gene encoding human parathyroid hormone (hPTH) has been cloned into this site, (e.g., into pLJ) in the following way: The pLJ plasmid was digested with BamHI and subsequently treated with the enzyme calf intestinal phosphatase. Following this, the linear vector was fractionated on argarose gel and purified, using glass beads. In addition, the BamHI fragment containing the human PTH gene was prepared from the plasmid described by Hendy et al., which contains a complete cDNA of human PTH cloned into pBR322. Hendy, G.N. et al., Proceedings of the National Academy of Sciences, USA, 78:7365-7369 (1981).

A sub fragment of the PTH cDNA, containing 17 bp of 5' untranslated, all coding and 31 bp of 3' untranslated sequence, was isolated by digesting the initial plasmid with DdeI and HinfI and isolating the 600bp fragment. The fragment was incubated with DNA polymerase in the presence of deoxynucleoside in phosphates, to fill in the recessed ends. BamHI linkers were ligated to the blunt ends with T₄ DNA ligase. An authentic BamHI restriction fragment was generated by digesting this ligation mixture with BamHI. This was then subcloned into the BamHI site of pBR322, which is the plasmid used as the source of hPTH in vector construction.

Equal quantities of the pLJ linear backbone and the BamHI PTH fragment were added together, in the presence of T4 DNA ligase. The resulting mixture was maintained under conditions appropriate for ligation of the two fragments. The ligation mixture was used to transform bacteria HB101, which were then plated onto agar containing kanamycin. Maniatis, T. et al., Molecular Cloning: A Laboratory Manual, Cold Spring Harbor Laboratory, p.p. 250-251, 504; Bolivar, F. and K. Backman, In: Methods in Enzymology, R. Wu (ed.), Vol. 68, Academic Press, N.Y. (1979). The resulting colonies were analyzed for the recombinant plasmid.

Parathyroid hormone is a polypeptide which has a role in the regulation of calcium in the body. Although the hPTH gene is present in human endothelial cells, it is not expressed in those cells at biologically significant levels. Endothelial cells capable of making a polypeptide hormone such as hPTH, or another substance not normally made by such cells at biologically significant levels, can be engrafted onto or implanted into an individual and serve as a continuous supply system for the hormone, or other substance.

The Psi am cells producing the recombinant virus construct which contained the hPTH-encoding DNA and DNA encoding a selectable marker (such as the neo gene), were used to produce a viral stock, as described above. The viral stock was harvested; endothelial cells to be transduced with the virus containing the hPTH gene were incubated with the stock. In this case, a selectable marker is used to identify and select for transduced endothelial cells by culturing on media containing G418. If the viral titer is sufficiently high, essentially all endothelial cells are infected and selection, using a selectable marker and appropriate media, is not needed.

The ability of endothelial cells transduced with the recombinant retrovirus having the hPTH gene to express the hPTH gene has been assessed in vitro as follows: The in vitro production of hPTH by endothelial cells established from a bovine aorta and infected with a virus containing the hPTH gene was assessed. The bovine aortic endothelial cells were derived from an explant from the aorta of a calf. The cells have a limited life span and are referred to as secondary cultures.

Bovine aortic endothelial cells were infected with virus and not selected in neomycin, as described above. Transduced bovine aortic endothelial cells were seeded onto 10 cm tissue culture dishes and grown to confluence. Fresh culture media (DME with 10% CS and penicillin and streptomycin) was then added; this point is referred to subsequently as time zero. At the end of 24 hours, the media was removed and the cells were assayed for the production of human PTH.

The aliquots were analyzed for the presence of hPTH using a radioimmunoassay (Nichols) which measures intact hPTH. The technique is described in Allegro™ Intact PTH/Immunoassay System for the Quantitative Determination of Human Intact Parathyroid Hormone in Serum, Nichols Institute Diagnostics, San Juan Capistrano, CA (36B-2170, Effective 7/86 Revised), the teachings of which are incorporated herein by reference. The assay has a sensitivity of approximately one nanogram/milliliter serum (ng/ml) and was shown to be specific for human PTH in that it does not cross react with calf PTH. The results of the experiments are reported as the production of hPTH, as measured by RIA, over time. Results are shown in Table I.

**TABLE I**

| Production of hPTH in Transduced BAE Cells | |
|---|---|
| Cell | PTH Production |
| Control BAE^{*} | 10 pg/10⁶/24h |
| Transduced BAE | 6.3ng/10⁶/24h |

| | |
|---|---|
| ^{*}BAE = bovine aortic endothelial | |

Endothelial cells from a bovine aorta transduced with DNA encoding hPTH according to the method of the present invention have been deposited at the American Type Culture Collection (Rockville, MD) under deposit number CRL9601, under conditions appropriate to meet the requirements of the Budapest Treaty.

### EXAMPLE 2 Production of Human LDL Receptor in Transduced Endothelial Cells

The cDNA for human LDL receptor (LDLR) was inserted into the pEM vector. The cDNA for LDLR was prepared for insertion into the pEM as follows. LDLR cDNA was excised from the vector pTZ1 (obtained from Dr. Goldstein, University of Texas Health Science Center) by digestion with HindIII. The 2.6 kb HindIII fragment containing all of the LDLR coding sequence was blunt ended with Klenow fragment and BclI oligonucleotide linkers (from NEB) were ligated to the cDNA with T4 DNA ligase. Finally, this ligation mixture was digested with an excess of the enzyme BclI and the fragment was fractionated on an agarose gel and purified. This was inserted into the BamHI cloning site of pEM as follows. pEM was digested with BamHI and the linearized plasmid was digested with calf intestinal phosphatase. Equal quantities of the linkered LDLR insert and pEM backbone were mixed and ligated with T4 DNA ligase. The ligation mixture was used to transform HB101 and ampicillin resistant colonies were analyzed for the appropriate retroviral vector. The pEM-LDLR vector was transfected into the Psi-am cell line and clones that produced high quantity of recombinant virus were identified. Viral stocks were used to infect cultures of bovine aortic endothelial cells as described for PTH.

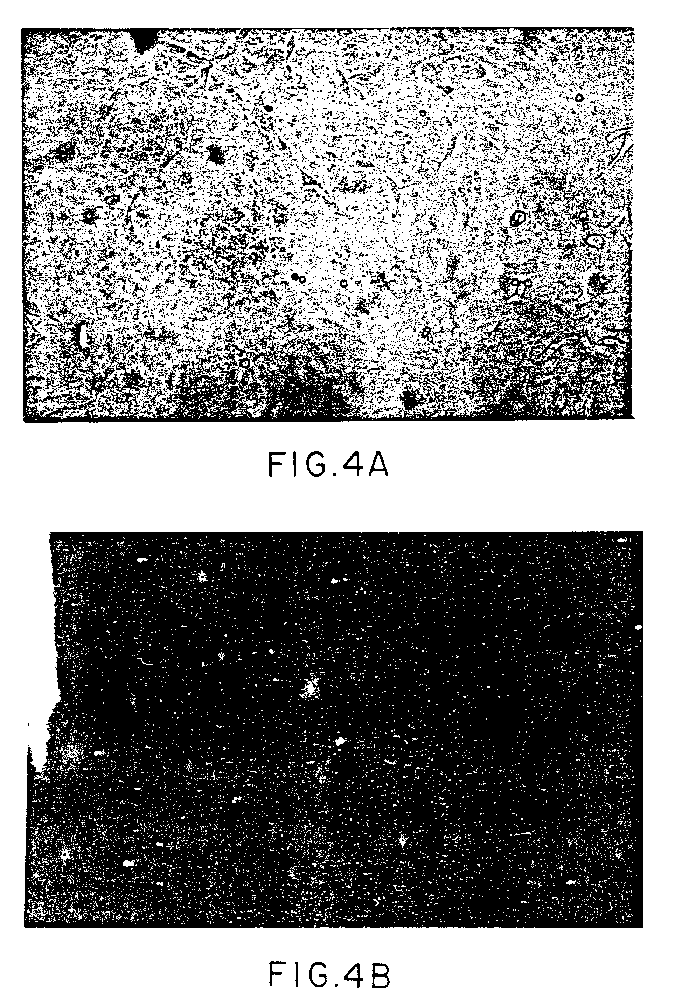

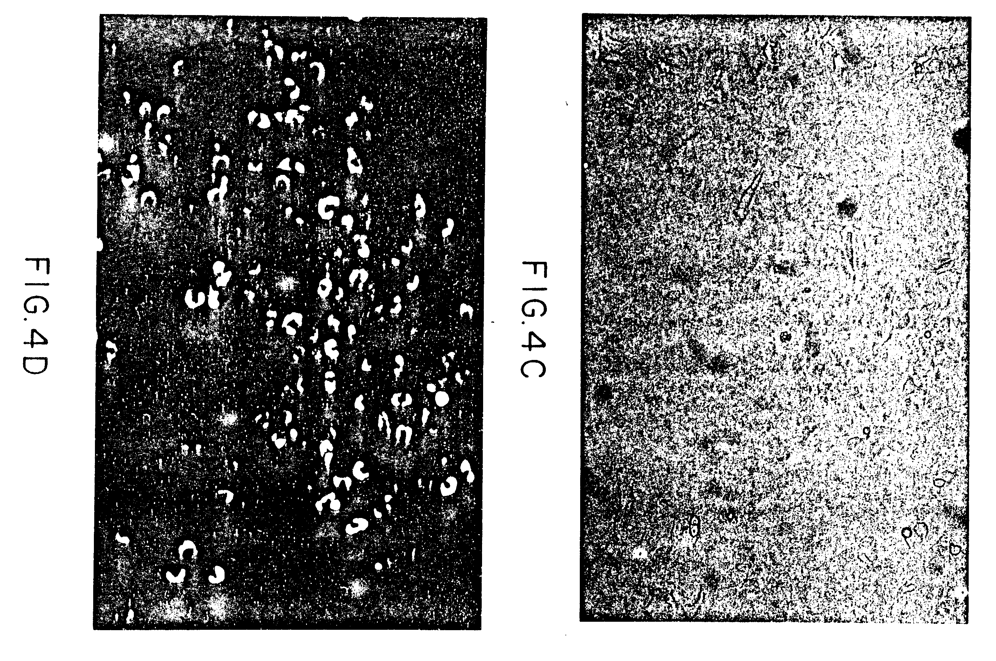

In vitro assessment of LDLR expression was carried out as follows: confluent plates of control or transduced bovine aortic endothelial cells were incubated with LDL that had been conjugated with a fluorescent label (obtained from Biomedical Tech Inc., Stoughton, MA) at a concentration of 10 ug/ml for approximately 8 hours. Following this, the cells were washed with PBS and fixed in 0.5% gluteraldehyde in PBS. They were then visualized under a fluorescent microscope for the uptake of fluorescent labeled LDL (*LDL). The results of this experiment are presented in Figure 4. Briefly, the level of endogenous LDLR is low, as evidenced by the relative lack of *LDL uptake in uninfected cultures (See 4B). However, in cultures infected with the LDLR virus, approximately 30% of all cells take up detectable quantities of *LDL, thereby documenting efficient expression of the exogenous LDLR (See 4D).

### EXAMPLE 3 Production of Beta-galactosidase in Transduced Endothelial Cells

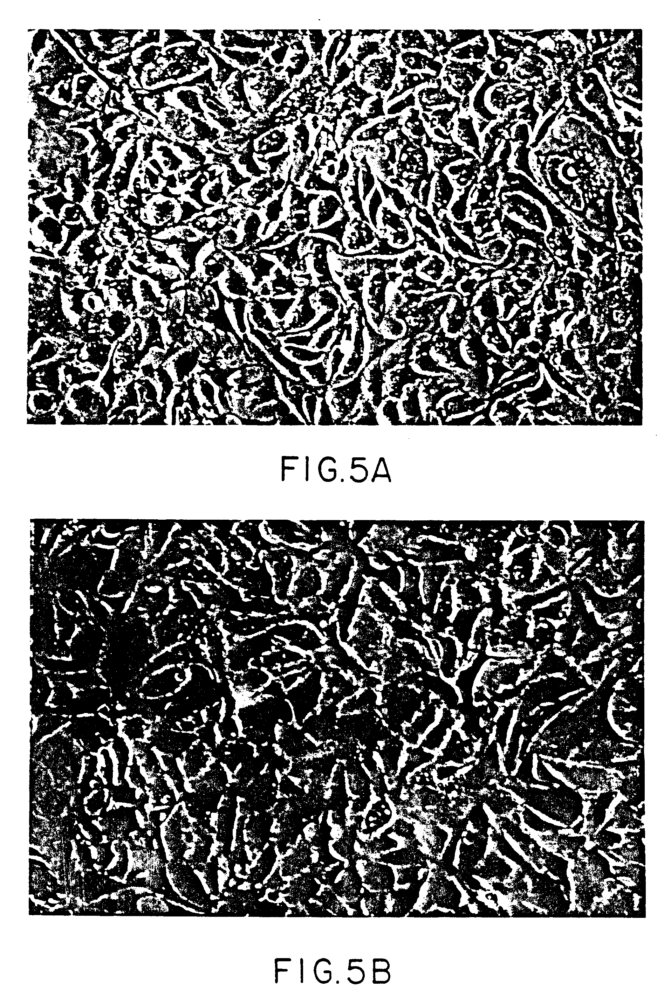

The gene endocing beta-galactosidase from E. coli was inserted into pLJ and this vector was transfected into the Psi-am cell line, resulting in production of high titer retroviral stocks. The construction of this vector and isolation of the producer cell line has been described by Price and co-workers. Price, J. et al., Proceedings of the National Academy of Sciences, USA, 84:156-160 (1987). Stocks of virus encoding the beta-galatosidase gene were used to infect bovine aortic endothelial cells, as described earlier. The infected cultures were analyzed for beta-galatosidase expression using an in situ histochemical stain. See Price et al. above. Cultures were analyzed before and after selection in G418. The retroviral vector used in this experiment expresses both beta-galactosidase and the neo gene which confers resistance to G418. The histochemical stain was performed as described by Price et al. Briefly, cell cultures were fixed in 0.5% gluteraldehyde in PBS for 5 minutes, washed with PBS and exposed to the reaction mixture for at least 12 hours. The reaction mixture contains a substrate for beta-galatosidase which, when hydrolyzed, turns blue and precipitates in the cell. As a result, any cell expressing the viral encoded beta-galactosidase will turn blue. The results of this experiment arc presented in Figure 5. No beta-galactosidase activity is detected in cultures that were not exposed to virus (Figure 5A); infected cultures demonstrate beta-galactosidase activity in about 30% of the cells (Figure 5B). These transduced cells are selected for by incubating them in the presence of G418.

### EXAMPLE 4 Production of Beta-galactosidase in Transduced Endothelial Cells on the Surface of Vascular Grafts Implanted Into Dogs

Endothelial cells were enzymatically harvested from external jugular veins of adult mongrel dogs which weighed 20-25 kg. Cells from each animal were divided into 2 aliquots; one to be infected with a replication defective retrovirus (see below) and the other to be mock infected. The enzymatically harvested cells were used to establish primary cultures. Endothelial cells were plated on to fibronectin coated flasks and maintained in M199 Medium supplemented with 5% plasma derived equine serum, penicillin, streptomycin, heparin and ECGF during two serial passages over a 10-14 day period.

During this time period, cells were exposed to fresh stocks of virus supplemented with polybrene (8 ug/ml) every 3 days (18 hr/exposure). At the end of the second passage, cells were harvested and aliquots were analyzed directly, cryopreserved, or used to seed 6 cm x 4 mm knitted dacron TM grafts (CR BARD, Billerica, MA) according to a modification of the 4-step method or Yates (0.75 x 10⁶ cells were added to the autologous blood during the second and third step). Animals were anesthetized and 6 cm segments of both carotid arteries were replaced with the seeded grafts as described. Each animal received an implant seeded with infected endothelial cells and a contralateral graft seeded with MOCK infected cells. Five weeks after implantation, the animals were anesthetized and the grafts were harvested and analyzed.

Replication-defective retroviruses with amphotropic host range were used to stably introduce a reporter gene into the genomic DNA of the endothelial cells. The lac Z gene was used as the reporter gene because its product of expression, beta-galactosidase, can be detected in situ through the use of enzyme histochemical assays that stain the cell's cytoplasm blue. (Example 3). Efficiency of retroviral infection was estimated by Southern analysis which detects proviral sequences and by the cytochemical stain for viral-expressed beta-galactosidase which detects infected cells in situ.

There were two recombinant retroviruses used in these studies. The BAG vector has been described previously by Price et al., Proceedings of the National Academy of Science USA, 84: 156-160 (1987). The BAG virus, containing the Lac Z-gene, expresses Beta-galactosidase from the 5' LTR (Long Terminal Repeat) and a selectable marker (Neo) from an SV40 derived promoter which confers resistance to kanamycin in prokaryotes and G418 in eukaryotes. Approximately 5-15% of the endothelial cells exposed to the BAG virus were infected (summarized in Table II); cultivation of these cultures in media supplemented with the aminoglycoside G418 effectively selected for transduced cells.

The BAL vector is derived from the previously described BA-LDLR vector except LDLR cDNA sequences were replaced with the coding sequences for the E. coli beta-galactosidase gene. The higher titer BAL virus, containing the Lac Z-gene, expresses beta-galactosidase from a promoter derived from the chicken beta-actin. Approximately 50% of the cells exposed to the BAL virus were transduced as measured by Southern analysis and by the in situ cytochemical stain for beta-galactosidase.

In Southern analysis, high molecular weight DNA was isolated and an aliquot (10 ug) was digested with Kpn I, fractionated on a 1% agarose gel, transferred to zetabind and probed with a 1200 bp Cla I/ECORI fragment that had been labeled to a high specific activity according to the method of Feinberg and Volt.

Cytochemical characterization of cultured endothelial cells was demonstrated by both phase contrast and fluorescent micrographs. Retrovirus-infected endothelial cells were analyzed at the time of seeding and after removal from the implanted graft for uptake of DIL-AC-LDL and for expression of viral-directed beta-galactosidase. Pre-seeded and post-implantation cells that had been inoculated with DIL-AC-LDL were assessed using phase contrast and fluorescent micrographs. Pre-seeded and post-implanted cells were also analyzed for the expression of viral directed beta-galactosidase.

At the time of seeding, the cultures were analyzed for endothelial cell specific function (i.e., uptake of acetylated LDL, and the presence of Von Willebrands factor) and for the presence of antigens specific for smooth muscle cells. These analyses indicated that more than 98% of the cells from each isolate were functioning endothelial cells.

The experimental system was a modification of a previously described dog model that has been used to study graft repopulation. Dogs 1-3 received implants that had been seeded with BAG-infected, unselected endothelial cells while dogs 4-7 initially received implants seeded with the BAL-infected, unselected endothelial cells. While these experiments were in progress, endothelial cells from dogs 4-7 were also infected with the BAG virus and expanded in culture in the presence or absence of G418, an aminoglycoside that selects for BAG-transduced cells. After 5 weeks, the grafts were explanted for analysis and new grafts were implanted in dogs 4-7 (referred to as dogs 4'-7'). Each of these dogs received a graft seeded with endothelial cells that were infected with BAG virus and selected in G418 and a contralateral graft seeded with BAG infected unselected endothelial cells. The second set of grafts were again explanted after 5 weeks.

Table II summarizes the results of these experiments. Animals 4' through 7' represent the second experiment performed on animals 4 through 7 (see text). BAG-U represents BAG infected endothelial cells unselected with G418 for transduced cells. BAG-S represents BAG infected endothelial cells selected with G418 for transduced cells. BAL represents BAL infected endothelial cells. MOCK represents MOCK infected cells. E is an indication of the efficiency of infection as measured by the beta-galactosidase stain. Patency of the graft after 5 weeks is indicated with a (Y) yes or (N) no. Coverage of the graft is an indication of the per cent of the surface repopulated as measured by scanning electron microscopy.

**Table II**

| Summary of Implantation Experiments | | | | | | | | |
|---|---|---|---|---|---|---|---|---|
| Animal+ | Experimental Graft | | | | Control Graft | | | |
| | Infection | | Explant | | Infection | | Explant | |
| | Virus | E | Patent | Coverage | Virus | E | Patent | Coverage |
| 1 | BAG-U | 15 | Y | 50-90 | MOCK | O | Y | 50-90 |
| 2 | BAG-U | 5 | Y | 50-90 | MOCK | O | Y | 50-90 |
| 3 | BAG-U | 5 | Y | 50-90 | MOCK | O | Y | 50-90 |
| 4 | BAL | 40-60 | N | - | MOCK | O | N | - |
| 5 | BAL | 40-60 | Y | O | MOCK | O | Y | 0 |
| 6 | BAL | 40-60 | Y | 50-90 | MOCK | O | Y | 50-90 |
| 7 | BAL | 40-60 | Y | 50-90 | MOCK | O | Y | 50-90 |
| 4' | BAG-S | 100 | Y | 50-90 | BAG-U | 10 | N | - |
| 5' | BAG-S | 100 | Y | 0 | BAG-U | 10 | N | - |
| 6' | BAG-S | 100 | Y | 0 | BAG-U | 10 | Y | 50-90 |
| 7' | BAG-S | 100 | Y | 50-90 | BAG-U | 10 | Y | 0 |

Analysis of the explanted grafts revealed that 18 of 22 remained patent after 5 weeks. Scanning electron microscopy demonstrated a lining of cells with endothelial-like morphology on the luminal surface of 14 of 18 patent grafts. When present, the endothelial cell lining was incomplete (50-90% of the total surface) and patchy in distribution with a paucity of cells seen along the peaks of the crimped dacron graft. A portion of each graft was fixed, stained for cells that express viral-derived beta-galactosidase and visualized through a high power dissecting microscope with a magnification of 750X. Each graft seeded with infected endothelial cells that remained patent and retained luminal cells did contain beta-galactosidase-positive cells on the lumen of the vessel. Contralateral grafts seeded with MOCK-infected endothelial cells never demonstrate positive staining cells.

In situ analysis of the grafts was performed for viral-transduced cells. Longitudinal sections of the genetically engineered implants were analyzed for cells that express viral-expressed beta-galactosidase. Grafts were cut longitudinally and a portion fixed in 0.5% gluteraldehyde for 5 minutes, washed in PBS three times, and incubated in x-gal solution for 2 hours and the luminal surface photographed with a Leitz dissecting microscope. The grafts were visualized enface and several interesting aspects of the seeding pattern were observed. The density of transduced cells was greatest in the deeper surfaces of the crimped graft. This was visualized under low power as rings of blue staining cells that line the crevices of the serrated surface and correlates with the variation in surface endothelial cell repopulation visualized by scanning electron microscopy. In addition there was no regional variation in the density or pattern of transduced cells with respect to proximity to the distal or proximal anastomosis. Finally, in each case the proportion of luminal cells which were positive for beta-galactosidase seemed qualitatively lower than the preparation of beta-galactosidase positive cells that were seeded.

Cells were enzymatically harvested from the luminal surface of portions of the grafts to permit a more detailed characterization. Primary cultures of cells were established and expanded in vitro for approximately 2-3 weeks prior to analysis. Genetically modified endothelial cells were identified in this population of cells by Southern analysis and by the cytochemical assay for viral-expressed beta-galactosidase. The majority of cells harvested from the graft and expanded in vitro retained differentiated endothelial function (less than 95%). However, the proportion of cells that expressed viral directed beta-galactosidase or contained proviral sequences was consistently diminished when compared to the cultures analyzed at the time of seeding. This disparity is due in part to the partial repopulation of grafts with endogenous cells by growth through interstices or from the anastomoses.

### Industrial Utility

This invention has industrial applicability in improving performance (e.g., duration, patency, thrombogenicity) of vascular grafts or implants, made of synthetic materials, which are often used in treating advanced atherosclerotic disease. It is also useful in providing hormones, enzymes and drugs to mammals, including humans, in need of such substances. For example, it can be used to provide a continuous supply of a hormone which otherwise would be administered on a intravenously, intramuscularly or subcutaneously. It is particularly valuable in providing such substances which are needed for extended periods of time.

### Equivalents

Those skilled in the art will recognize, or be able to ascertain using no more than routine experimentation, many equivalents to the specific embodiments of the invention described specifically herein. Such equivalents are intended to be encompassed in the scope of the following claims.

## Claims

1. Endothelial cells (e.g. human endothelial cells) for use in therapy, the cells containing stably introduced genetic material encoding a product of interest, the cells being capable of expressing the genetic material to produce the encoded product.

2. Use of endothelial cells (e.g. human endothelial cells) for the manufacture of a medicament for use in therapy, the cells containing stably introduced genetic material encoding a product of interest, and being capable of expressing the genetic material to produce the encoded product.

3. Endothelial cells according to claim 1 or use according to claim 2 wherein the introduced genetic material is DNA or RNA which; (a) does not normally occur in endothelial cells, (b) normally occurs in endothelial cells but is not expressed in them at biologically significant levels, (c) normally occurs in endothelial cells and has been modified so that it is expressed in endothelial cells, or (d) can be modified to be expressed by endothelial cells, alone or in any combination thereof.

4. Endothelial cells or use according to any one of claims 1 to 3, wherein the introduced genetic material encodes; (a) a hormone, e.g. human parathyroid hormone, (b) an enzyme, for example an intracellular enzyme, e.g. beta-galactosidase, (c) a drug, (d) a (dominant) selectable marker, for example encoding antibiotic resistance, e.g. a neo gene, (e) a protein or polypeptide which is secreted from the cells, or (f) a membrane receptor, e.g. the low density lipoprotein receptor.

5. Endothelial cells or use according to claim 4, for providing the hormone, enzyme or drug to the body by; (a) introduction of the endothelial cells into the body or (b) application of the endothelial cells to a surface of the body.

6. Endothelial cells or use according to any one of claims 1 to 5 wherein the genetic material encoding the product of interest has been stably incorporated by transduction.

7. Endothelial cells or use according to claim 6 wherein the transduction is via a retrovirus having a recombinant genome comprised of; (a) genetic material encoding the product of interest, (b) the long terminal repeat sequences, tRNA binding site and Psi packaging site derived from an amphotropic retrovirus; and (c) at least one promoter of eukaryotic origin which can, for example, be modulated by an external cue.

8. Endothelial cells or use according to any one of claims 1 to 7 wherein the genetic material encodes a thrombolytic protein or portion thereof, for example tissue plasminogen activator or streptokinase, for application to a synthetic prosthetic vessel to reduce the thrombogenicity thereof.

9. Endothelial cells or use according to any one of claims 1 to 7 wherein the genetic material encodes an endothelial cell mitogen or an endothelial cell growth factor for application, under conditions appropriate for endothelial cell proliferation, to the inner surface of a prosthetic vessel to produce a confluent layer of endothelial cells lining said inner surface.

10. Endothelial cells or use according to any one of claims 1 to 9 for seeding the inner surface of a prosthetic vessel at subconfluent levels to produce a lining on the inner surface of said vessel having endothelial cells which express the introduced genetic material encoding a product of interest.

11. Endothelial cells or use according to any one of claims 1 to 7 wherein the genetic material encodes a membrane receptor which is capable of binding a ligand, the cells being for seeding onto a surface of a prosthetic vessel bearing the ligand, whereby (under conditions appropriate for binding of the ligand and the membrane receptor) binding of the endothelial cells to the surface of the prosthetic vessel is enhanced.

12. Endothelial cells or use according to any one of claims 1 to 7 wherein the genetic material encodes a product which inhibits smooth muscle cell growth, for inhibiting the growth of smooth muscle cells in a synthetic vascular implant.

13. A method of making the endothelial cells according to any one of claims 1 to 12, comprising the steps of; (a) contacting cultured endothelial cells with media containing an infectious retrovirus having a recombinant genome comprised of the genetic material of interest and (b) maintaining the cultured endothelial cells and the media containing the infectious recombinant retrovirus under conditions appropriate for infection of the endothelial cells by the recombinant retrovirus.

14. A method of making endothelial cells according to any one of claims 1 to 12, comprising the steps of; (a) transducing cultured endothelial cells with genetic material encoding a product of interest, and (b) culturing the transduced endothelial cells under conditions appropriate for their growth.

15. A lined prosthetic vessel comprising the endothelial cells of claim 10, for implantation into a mammal and expression of the incorporated genetic material in the mammal.

## Patentansprüche

1. Endothelzellen (z.B. menschliche Endothelzellen) zur Verwendung in der Therapie, wobei die Zellen stabil eingeführtes genetisches Material enthalten, das für ein gewünschtes Produkt kodiert, und wobei die Zellen in der Lage sind, das genetische Material zur Herstellung des kodierten Produkts zu exprimieren.

2. Verwendung von Endothelzellen (z.B. menschlichen Endothelzellen) zur Herstellung eines Arzneistoffs zur Verwendung in der Therapie, wobei die Zellen stabil eingeführtes genetisches Material enthalten, das für ein gewünschtes Produkt kodiert, und wobei sie in der Lage sind, das genetische Material zur Herstellung des kodierten Produkts zu exprimieren.

3. Endothelzellen nach Anspruch 1 oder deren Verwendung nach Anspruch 2, bei denen das eingeführte genetische Material DNA oder RNA ist, die jeweils allein oder in irgendeiner Kombination davon
(a) normalerweise nicht in Endothelzellen vorkommt,
(b) normalerweise in Endothelzellen vorkommt, jedoch in diesen nicht in biologisch signifikanten Mengen exprimiert wird,
(c) normalerweise in Endothelzellen vorkommt und so modifiziert worden ist, daß sie in Endothelzellen exprimiert wird oder
(d) modifiziert werden kann, so daß sie von Endothelzellen exprimiert wird.

4. Endothelzellen oder deren Verwendung nach einem der Ansprüche 1 bis 3, bei denen das eingeführte genetische Material kodiert für
(a) ein Hormon, z.B. menschliches Parathyroidhormon,
(b) ein Enzym, z.B. ein intrazelluläres Enzym, z.B. β-Galactosidase,
(c) einen Arzneistoff,
(d) einen (dominanten) Selektionsmarker, der z.B. für Antibiotikaresistenz kodiert, z.B. ein neo-Gen,
(e) ein Protein oder Polypeptid, das von den Zellen sekretiert wird, oder
(f) einen Membranrezeptor, z.B. den Low-density-Lipoproteinrezeptor.

5. Endothelzellen oder deren Verwendung nach Anspruch 4 zur Bereitstellung des Hormons, Enzyms oder Arzneistoffs für den Körper durch
(a) Einführen der Endothelzellen in den Körper oder
(b) Applikation der Endothelzellen auf eine Oberfläche des Körpers.

6. Endothelzellen oder deren Verwendung nach einem der Ansprüche 1 bis 5, bei denen das genetische Material, das für das gewünschte Produkt kodiert, durch Transduktion stabil inkorporiert wurde.

7. Endothelzellen oder deren Verwendung nach Anspruch 6, bei denen die Transduktion mittels eines Retrovirus erfolgt, der ein rekombinantes Genom besitzt, umfassend
(a) genetisches Material, das für das gewünschte Produkt kodiert,
(b) die Long-terminal-repeat-Sequenzen, die tRNA-Bindungssequenz und die Psi-Verpackungssequenz, abgeleitet von einem amphotropen Retrovirus und
(c) mindestens einen Promotor eukaryontischen Ursprungs, der z.B. durch ein externes Signal reguliert werden kann.

8. Endothelzellen oder deren Verwendung nach einem der Ansprüche 1 bis 7, bei denen das genetische Material für ein thrombolytisches Protein oder einen Teil davon kodiert, z.B. Gewebe-Plasminogenaktivator oder Streptokinase, zur Applikation auf ein synthetisches prothetisches Gefäß zur Verminderung seiner Thrombogenizität.

9. Endothelzellen oder deren Verwendung nach einem der Ansprüche 1 bis 7, bei denen das genetische Material für ein Mitogen oder einen Wachstumsfaktor für Endothelzellen kodiert, zur Applikation auf die innere Oberfläche eines prothetischen Gefäßes unter Bedingungen, die für die Proliferation von Endothelzellen geeignet sind, um eine konfluente Schicht von Endothelzellen herzustellen, die die innere Oberfläche bedeckt.

10. Endothelzellen oder deren Verwendung nach einem der Ansprüche 1 bis 9 zum Aussäen auf die innere Oberfläche eines prothetischen Gefäßes bei subkonfluenter Zellzahl, zur Herstellung einer bedeckenden Schicht auf der inneren Oberfläche dieses Gefäßes, die Endothelzellen aufweist, die das für ein gewünschtes Produkt kodierende eingeführte genetische Material exprimieren.

11. Endothelzellen oder deren Verwendung nach einem der Ansprüche 1 bis 7, bei denen das genetische Material für einen Membranrezeptor kodiert, der in der Lage ist, einen Liganden zu binden, wobei die Zellen zum Aussäen auf eine Oberfläche eines den Liganden tragenden Gefäßes geeignet sind, und wobei (unter Bedingungen, die für die Bindung des Liganden und des Membranrezeptors geeignet sind) die Bindung der Endothelzellen an die Oberfläche des prothetischen Gefäßes verstärkt wird.

12. Endothelzellen oder deren Verwendung nach einem der Ansprüche 1 bis 7, wobei das genetische Material für ein Produkt kodiert, das das Wachstum glatter Muskelzellen inhibiert, zur Inhibition des Wachstums glatter Muskelzellen in einem synthetischen Gefäßimplantat.

13. Verfahren zur Herstellung der Endothelzellen nach einem der Ansprüche 1 bis 12, umfassend
(a) Kontaktieren der kultivierten Endothelzellen mit Medien, die ein infektiöses Retrovirus mit einem rekombinanten Genom enthalten, das das gewünschte genetische Material umfaßt, und
(b) das Inkubieren der kultivierten Endothelzellen und der Medien, die das infektiöse rekombinante Retrovirus enthalten, unter Bedingungen, die für die Infektion der Endothelzellen durch das rekombinante Retrovirus geeignet sind.

14. Verfahren zur Herstellung der Endothelzellen nach einem der Ansprüche 1 bis 12, umfassend
(a) das Transduzieren der kultivierten Endothelzellen mit genetischem Material, das für ein gewünschtes Produkt kodiert, und
(b) das Kultivieren der transduzierten Endothelzellen unter Bedingungen, die für ihr Wachstum geeignet sind.

15. Ein mit einer Schicht bedecktes prothetisches Gefäß, umfassend die Endothelzellen von Anspruch 10 zur Implantierung in einen Säuger, und Expression des inkorporierten genetischen Materials in dem Säuger.

## Revendications

1. Cellules endothéliales (par exemple cellules endothéliales humaines) destinées à un usage thérapeutique, lesdites cellules contenant un matériel génétique introduit de manière stable codant pour un produit intéressant et étant capables d'exprimer le matériel génétique de manière à produire le produit codé.

2. Utilisation de cellules endothéliales (par exemple de cellules endothéliales humaines) pour fabriquer un médicament à usage thérapeutique, lesdites cellules contenant un matériel génétique introduit de manière stable codant pour un produit intéressant et étant capables d'exprimer le matériel génétique de manière à produire le produit codé.

3. Cellules endothéliales selon la revendication 1 ou utilisation selon la revendication 2, où le matériel génétique introduit est de l'ADN ou de l'ARN qui: (a) ne se trouve normalement pas dans les cellules endothéliales; (b) se trouve normalement dans les cellules endothéliales mais n'est pas exprimé dans ces cellules à un taux significatif au plan biologique; (c) se trouve normalement dans les cellules endothéliales et a été modifié de façon à pouvoir être exprimé par les cellules endothéliales ou (d) peut être modifié pour être exprimé par des cellules endothéliales, seul ou sous forme d'une combinaison quelconque.

4. Cellules endothéliales ou utilisation selon l'une des revendications 1 à 3, où le matériel génétique introduit code: (a) pour une hormone, par exemple la parathormone humaine; (b) pour une enzyme, par exemple une enzyme intracellulaire comme la bêta-galactosidase; (c) pour un médicament; (d) pour un marqueur susceptible d'être sélectionné (dominant), codant pour par exemple un facteur de résistance à un antibiotique, comme un gène neo; (e) une protéine ou un polypeptide sécrété à l'extérieur des cellules ou (f) un récepteur membranaire, par exemple le récepteur des lipoprotéines de basse densité.

5. Cellules endothéliales ou utilisation selon la revendication 4, destinées à fournir l'hormone, l'enzyme ou le médicament à l'organisme par (a) introduction des cellules endothéliales dans l'organisme ou (b) application des cellules endothéliales à la surface de l'organisme.

6. Cellules endothéliales ou utilisation selon l'une des revendications 1 à 5, où le matériel génétique codant pour le produit intéressant a été incorporé de manière stable par transduction.

7. Cellules endothéliales ou utilisation selon la revendication 6, où la transduction est effectuée via un rétrovirus possédant un génome recombinant constitué de : (a) un matériel génétique codant pour le produit intéressant; (b) les longues séquences répétitives terminales, le site de liaison de l'ARNt et le site d'encapsidation Psi dérivés d'un rétrovirus amphotrope; et (c) au moins un promoteur d'origine eucaryote qui peut, par exemple être modulé par un signal externe.

8. Cellules endothéliales ou utilisation selon l'une des revendications 1 à 7, où le matériel génétique code pour une protéine thrombolytique ou une partie de cette demière, par exemple l'activateur du plasminogène des tissus ou la streptokinase, pour l'application sur un vaisseau prosthétique pour en réduire la thrombogénicité.

9. Cellules endothéliales ou utilisation selon l'une des revendications 1 à 7, où le matériel génétique code pour un mitogène des cellules endothéliales ou un facteur de croissance des cellules endothéliales pour l'application, dans des conditions appropriées à la prolifération des cellules endothéliales, sur la surface interne d'un vaisseau prosthétique de manière à tapisser ladite surface interne d'une couche confluante de cellules endothéliales.

10. Cellules endothéliales ou utilisation selon l'une des revendications 1 à 9, pour l'ensemencement de la surface interne d'un vaisseau prosthétique à un niveau sub-confluant de manière à tapisser la surface interne dudit vaisseau avec des cellules endothéliales qui expriment le matériel génétique introduit codant pour un produit intéressant.

11. Cellules endothéliales ou utilisation selon l'une des revendications 1 à 7, où le matériel génétique code pour un récepteur membranaire susceptible de se lier à un ligand, les cellules étant destinées à être ensemencées à la surface d'un vaisseau prosthétique portant le ligand, de manière à (dans des conditions appropriées à la liaison du ligand et du récepteur membranaire) à accroître la liaison des cellules endothéliales à la surface du vaisseau prosthétique.

12. Cellules endothéliales ou utilisation selon l'une des revendications 1 à 7, où le matériel génétique code pour un produit qui inhibe la prolifération des cellules musculaires lisses, de manière à inhiber la prolifération de cellules musculaires lisses dans un implant vasculaire synthétique.

13. Procédé de préparation de cellules endothéliales selon les revendications 1 à 12, comprenant les étapes consistant à: (a) mettre en contact des cellules endothéliales en culture avec un milieu contenant un rétrovirus infectieux possédant un génome recombinant contenant le matériel génétique intéressant et (b) maintenir les cellules endothéliales en culture et le milieu contenant un rétrovirus infectieux dans des conditions appropriées à l'infection des cellules endothéliales par le rétrovirus recombinant.

14. Procédé de préparation de cellules endothéliales selon les revendications 1 à 12, contenant les étapes consistant à: (a) transduire des cellules endothéliales en culture avec un matériel génétique codant pour un produit intéressant, et (b) cultiver les cellules endothéliales transduites dans des conditions appropriées à leur prolifération.

15. Récipient prosthétique tapissé comprenant les cellules endothéliales de la revendication 10, destiné à être implanté dans un mammifère et à exprimer le matériel génétique incorporé dans le mammifère.
